# EUROPEAN PATENT SPECIFICATION

(11) **EP 3 494 939 B1**
(45) Date of publication and mention of the grant of the patent: **20.07.2022**
(21) Application number: 18207776.8
(22) Date of filing: 22.11.2018
(51) Int. Cl.: A61F 7/10, A61F 7/02, A61F 7/00

(54) **TOPICAL COOLING DEVICE**
TOPISCHE KÜHLVORRICHTUNG
DISPOSITIF DE REFROIDISSEMENT TOPIQUE

(30) Priority: 06.12.2017 US 201762595195 P
(43) Date of publication of application: 12.06.2019
(73) Proprietor: Becton, Dickinson and Company, Franklin Lakes, NJ 07417-1880 (US)
(72) Inventor: Gildea, Christopher, Spartan NJ 07417 (US); Limaye, Amit, Wayne NJ 07470 (US)
(74) Representative: dompatent von Kreisler Selting Werner - Partnerschaft von Patent- und Rechtsanwälten mbB

(56) References cited:
- WO-A1-01/45633
- WO-A1-01/51409
- WO-A1-2017/096212
- WO-A2-2004/012575
- US-A1- 2011 040 361
- US-A1- 2012 213 571

## Description

### Field of the Invention

The present invention is directed to a device for cooling, desensitizing, and/or numbing the surface of the skin. A device is disclosed for desensitizing, cooling, and/or numbing a target area of the skin prior to an injection by a medical device such as a needle or cannula.

### Background

Insulin and other injectable medications are commonly delivered by drug delivery pens, whereby a disposable pen needle hub is attached to the pen to facilitate drug container access and allow fluid to pass from the container through the needle into the patient.

The use of pen needles and other delivery devices, such as syringes, that include a needle or cannula can cause pain and discomfort to the patient. Various methods have been proposed to reduce the pain and discomfort to the user, such as by contacting the injection target site with ice or a cold object. While these methods can provide some relief, there is a need for improved devices and methods for reducing the pain and discomfort to the skin of the patient during an injection by a needle or cannula.

Various pen needle delivery devices are known in the art for dispensing the substance to the patient. The delivery devices often use a disposable needle hub having a cannula or needle extending from a patient end of the hub for inserting into the patient. A non-patient end of the hub is coupled to the pen delivery device for delivering the substance to the patient.

The needle hub assembly is often packaged in a container containing several loose needle hubs. A needle hub is selected from the package and attached to the pen needle delivery device for injecting the patient and then removed to be discarded. The needle hub package includes an outer cover that encloses the needle hub and a removable seal that is peeled from the outer cover to open the cavity so that the needle hub can be removed. The needle hub can have threaded non-patient end that is threaded onto the delivery device. The delivery device with the attached needle hub is then removed from the outer cover. An inner needle shield is attached to the needle hub to cover the cannula until the device is ready for use. The shield is removed to expose the cannula for use to deliver the substance to the patient. After use, the needle hub can be inserted back into the outer cover to enclose the exposed cannula. The pen delivery device is separated from the needle hub leaving the needle hub within the outer cover.

Existing pen needle assemblies are disclosed in U.S. Patent Application Publication Nos. 2006/0229562 to Marsh et al. and 2007/0149924 to R. Marsh, US2011040361 is also considered relevant prior art.

Although the prior devices have been suitable for the intended use, there is a continuing need in the industry for improved devices for use a pen needle and delivery devices.

### Summary

A cooling device is provided for contacting and cooling the surface of the skin to provide a desensitizing and numbing effect on the surface of the skin prior to injection or penetration of the skin by a delivery device. The cooling device is able to cool the surface of the skin to a sufficiently low temperature to provide a desensitizing and numbing effect prior to penetration by a medical device, such as a needle or cannula to reduce the pain and/or discomfort associated with the insertion of a needle or cannula. The cooling device is configured to contact and conform to the surface of the skin and cool a delivery target area on the skin where the target area is pierced by a needle, cannula or other medical device. The cooling device reduces the temperature of the skin in the target area to numb or desensitize the target area without freezing or damaging the skin in the target area.

The cooling device can be included with a kit, package, or assembly that includes an injection delivery device, such as a pen needle, infusion pump, or other injection apparatus that includes a cannula or needle. The cooling device has a contact area with a size and dimension for providing a desensitizing and/or numbing effect on the target area of the surface of the skin of the patient to reduce the discomfort associated with the penetration of the needle or cannula.

The cooling device in one embodiment includes an internal cavity containing a refrigerant medium, cooling material, or cooling composition, such as a refrigerant gel, that can be cooled or frozen prior to use and can be stored in a cool or frozen state for an extended period of time. The device has at least one contact surface that can be positioned against and directly contacting the target area of the skin of the patient to desensitize and cool the surface of the skin and provide the desensitizing, cooling, and/or numbing effect. The refrigerant medium can cool the surface of the skin to a temperature of 0-15°C and typically about 2-10°C. The cooling device cools the target area of the skin to a temperature to provide a numbing effect that does not cause injury or extreme discomfort to the skin.

In one embodiment, the cooling device includes a container having an open end and an internal cavity and a thermally transmitting member forming a closure for closing the open end of the container. A refrigerant medium, cooling liquid or cooling composition is contained in the cavity of the container and in contact with an inner surface of the closure. The thermally transmitting member is made of a heat transmitting or thermally conducting material that enables the refrigerant medium to cool the skin that contacts the thermally transmitting member to a temperature of about 2°C to 10°C. Refrigerant medium in one embodiment is in direct contact with an inner surface of the heat transmitting material.

The cooling device in one embodiment is sufficiently small to be stored in the pocket or package of the injection device. In one embodiment, the cooling device has a generally cylindrical shape with a length of about 2-3 inches (5.08 - 7.62 cm) and diameter of about 0.5 to 1 inch (1.27 - 2.54 cm ) . The thermally transmitting member has a sink contact area with a diameter of about 0.5 to 1 inch (1.27- 2.54 cm) .

The container of the cooling device can be made of a thermally insulating material and constructed to maintain the refrigerant medium in a frozen, cooled, or chilled state for an extended period of time. The container can be made of thermally insulating plastic material or metal that can include at least one thermally insulating layer made of an insulating material. The insulating material can be closed cell foam or open cell foam, such as a polyurethane or polystyrene.

In one embodiment, the container has an inner wall and an outer wall defining a cavity between the inner and outer walls. An insulating material, such as polymer foam, can be injected and/or molded between the inner and outer walls to provide the insulating properties of the container. In other embodiments, the cavity between the inner and outer walls can be filled with an insulating gas or air. In further embodiments, the cavity between the inner and outer walls can under a vacuum or reduced pressure to reduce the heat transmission between the inner and outer walls. The surface of the inner wall and the outer wall can include a heat reflective surface, film, or coating to inhibit the transmission of radiant heat into or out of the container.

In another embodiment, the cooling device includes a thermally insulating container with an open end and an internal cavity. A closure for the container can be a bulb-like member forming a bladder containing a refrigerant medium within the cavity of the container where a portion of the bladder is accessible through the open end of the container. The refrigerant medium or cooling composition in one embodiment can be a refrigerant liquid such as a hypertonic aqueous solution that can be frozen and provide a cooling effect for an extended period of time. The bladder can be made of a flexible polymer that is able to contain the refrigerant medium for repeated freezing and thawing cycles. The bladder can have a thickness sufficient to contain the refrigerant medium and provide sufficient heat transmission to cool the skin of a patient on contact with the surface of the bladder. The bladder can be made of a polymer, such as polyurethane, that can remain flexible at temperatures below 10°C and typically below 0°C.

The refrigerant medium can be a suitable liquid or gel that can be frozen and reused to provide a cooling, freezing, and desensitizing effect when contacting the skin to reduce the pain and discomfort during an injection. The refrigerant medium can be a gel or liquid. The refrigerant medium can be an aqueous mixture of propylene glycol, hydroxyethylcellulose, sodium polyacrylate, silica gel and the like. In other embodiments, the refrigerant medium can be an aqueous solution of sodium chloride and/or calcium chloride.

The bladder in one embodiment is received within the cavity of the thermally insulated container with an end portion projecting from the open end of the container a distance for contacting the skin of the patient during use. An insulating lid can be coupled to the open end of the container. The bladder can occupy substantially the entire internal volume of the container. In one embodiment, an air gap can be provided in a portion of the cavity of the container to accommodate for expansion during freezing of the refrigerant medium.

In another embodiment, the container includes a side wall, a bottom wall, an open top end, and closure member coupled to the container to close the open top end of the container. The container encloses a refrigerant medium that can be frozen and stored in a frozen state for an extended period of time. The closure member can be a flexible polymer that is able to cool the skin when the closure contacts the skin of the patient.

The objects, advantages, and salient features of the invention will become apparent from the following detailed description, which, taken in conjunction with the annexed drawings, discloses exemplary embodiments of the invention.

### Brief Description of the Drawings

The above benefits and other advantages of the various embodiments of the present invention will be more apparent from the following detailed description of exemplary embodiments of the present invention and from the accompanying figures, in which:
Fig. 1 is a side view of a container and lid in one embodiment;
Fig. 2 is a side view of Fig. 1 showing the lid separated from the container;
Fig. 3 is a cross-sectional view of the container of Fig. 1;
Fig. 4 is a cross-sectional view of the container in another embodiment;
Fig. 5 is a cross-sectional view of the container and lid in a further embodiment showing the insulating space between an inner and outer wall of the container;
Fig. 6 is a cross-sectional view of the container in another embodiment;
Fig. 7 is a cross-sectional view of the container in a further embodiment;
Fig. 8 is cross-sectional view of the container in another embodiment;
Fig. 9 is a cross-sectional view of the container in a further embodiment; and
Fig. 10 is a cross-sectional view of the container of Fig. 9 showing the bladder in the extended position.

Throughout the drawings, like reference numbers will be understood to refer to like parts, components, and structures.

### Detailed Description of the Exemplary Embodiments

Reference to embodiments of the present invention are illustrated in the accompanying drawings, wherein like reference numerals refer to like elements throughout. The embodiments described herein exemplify, but do not limit, the present invention by referring to the drawings. The exemplary embodiments are presented in separate descriptions, although the individual features and construction of these embodiments can be combined in any number of ways to meet the therapeutic needs of the user. The embodiments and/or elements can be combined with one another to form various additional embodiments not specifically disclosed, as long as the embodiments do not contradict one another.

It will be understood by one skilled in the art that this disclosure is not limited in its application to the details of construction and the arrangement of components set forth in the following description or illustrated in the drawings. The embodiments herein are capable of of being modified, practiced or carried out in various ways. It will be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Unless limited otherwise, the terms "connected," "coupled," and "mounted," and variations thereof herein are used broadly and encompass direct and indirect connections, couplings, and mountings. In addition, the terms "connected" and "coupled" and variations thereof are not limited to physical or mechanical connections or couplings. Further, terms such as up, down, bottom, and top are relative, and are employed to aid illustration, but are not limiting.

A cooling device is provided for cooling the surface of the skin of a patient to provide a temporary desensitizing or numbing effect to the skin immediately or shortly before the insertion of a needle, cannula or other medical device into the skin. The cooing device can be used alone or in combination with a delivery device such as a kit, package, or assembly.

The cooling device is primarily intended for use with a medical device, such as a delivery device, where a needle or cannula of the delivery device penetrates the skin for delivery of a substance, such as a pharmaceutical, or for withdrawing a fluid from the patient. Examples of a delivery device can include a pen needle, infusion pump, syringe, patch pump, lancet, catheter, or other injection or delivery device. The cooling device is also suitable for use in intravenous drug delivery devices and phlebotomy applications. The medical device can also be a catheter, central line ports, sub-surface ports that can be accessed for drug delivery, such as in chemotherapy applications. The delivery device in one embodiment is a pen needle commonly used delivery of insulin where it is necessary for the patient to introduce the needle into the skin to a controlled depth for the insulin delivery.

The cooling device contains a refrigerant medium or cooling medium that is re-usable for multiple uses. In one embodiment, the cooling device includes a refrigerant medium where the device can be placed in a freezer to freeze the refrigerant medium until ready for use. As used herein, a freezer refers to a device or apparatus that cools below 0°C and is able to cool the refrigerant medium below the freezing point of the refrigerant medium. The cooling device is able to keep the refrigerant medium in a frozen, cold, or chilled state for several hours to provide a useful life to the device. The cooling device in one embodiment is able to maintain the cold temperature for 6 to 8 hours during normal use. The refrigerant medium can be cooled to a temperature of a standard freezer to a temperature at or below -20°C. The cooling device is sufficiently insulated to maintain the temperature of the refrigerant medium at or below about 5°C and preferably at or below about 2°C for 6-8 hours when stored at room temperature.

In one embodiment, the cooling device has a sufficiently small size that the device is able to fit within the pocket of the user or can be retained within the package or container that stores the pen needle and supplies until ready for use. The cooling device in one embodiment can have a substantially cylindrical shape with a length of about 2-3 inches (5.08 - 7.62 cm) and a width or diameter of about 0.5 to 1 inch (1.25 - 2.54 cm) although other sizes and dimensions can be used as needed.

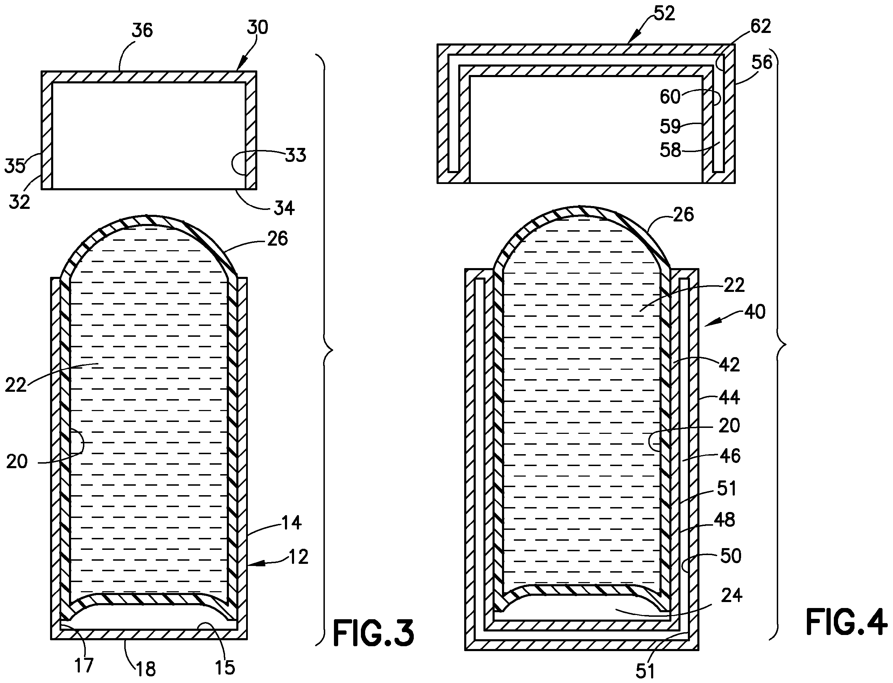

Referring to the drawings, the cooling device 10 includes a container 12 having a side wall 14, an open end 16, and a closed bottom end formed by a bottom wall 18. In the embodiment shown, the container 12 has a substantially cylindrical shape although other shapes can be used. The container 12 is made of a thermally insulating material that is able to withstand repeated freezing and thawing cycles and retain the refrigerant medium in a cooled or frozen state. In one embodiment, the container is made from a plastic material with a sufficient thickness and strength to retain the refrigerant medium in a frozen or cooled state for an extended period of time. The container 12 can be made of polymer foam and can include a heat reflective surface or coating 15 on an inner surface of the container. In the embodiment shown in Fig. 3, the heat reflective surface 15 is formed on the bottom wall 18 and the side wall 14. The outer surface of the container can also include a heat reflective coating 17 to inhibit heat conduction and reflect thermal radiation into or out of the container and the refrigerant medium.

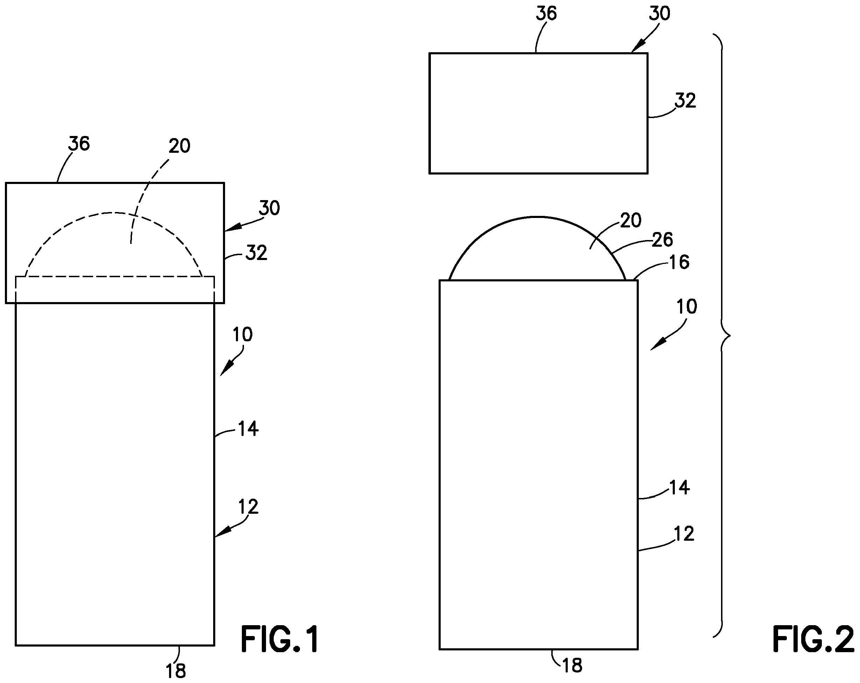

In the embodiment shown in Figs. 1-3, the cooling device 10 includes a thermally transmitting member functioning as a closure to enclose a cooling composition referred to as a refrigerant medium 22 within the container 12. In the embodiment shown, the thermally transmitting member closing the container is an inner container such as a flexible bladder 20 that contains and encloses the refrigerant medium 22. The bladder 20 forms an enclosure for the refrigerant medium 22 and is positioned within the cavity of the container 12 as shown in Fig. 2. The bladder 20 can be made of a flexible polymer that can conform to the inner configuration of the container 12. The bladder 20 is made of a polymer material that is able to withstand the freezing and thawing of the refrigerant medium and retain the refrigerant medium without leakage during the normal use of the device. In one embodiment, an air gap 24 is provided between the bladder 20 and the bottom wall 18 of the container to allow for expansion of the refrigerant medium during freezing. In other embodiments, an air gap or space can be provided in the bottom portion of the bladder 20 to allow for the expansion of the bladder 20 and the refrigerant medium 22 during freezing as shown in Fig. 2.

The bladder 20 in one embodiment is flexible to conform to the inner surfaces of the container and to conform to the contour of the skin of the patient when contacted with and pressed against the skin of the patient. In one embodiment, the bladder 20 can be adhesively attached to the walls 14 of the container 12. The bladder 20 has a thickness to contain the refrigerant medium while allowing sufficient heat transmission through the bladder to cool the skin of the patient. The bladder 20 forms a closed, self-contained unit for the refrigerant medium 22 to reduce the risk of leakage. The bladder is typically a flexible polymeric material. The bladder 20 can be fixed in the cavity of the container or can be removable from the container.

The container in the embodiment shown has a substantially cylindrical side wall with an open end complementing the inner dimension of the side wall. The bladder 20 occupies a substantial portion of the inner cavity of the container 12 to maximize the volume of the refrigerant medium 22 relative to the dimensions of the container. The side wall 14 covers and encloses a substantial portion of the bladder 20 to thermally insulate the bladder 20 during handling of the device.

In the embodiment shown, the bladder 20 has an end contact portion 26 forming a skin contact area for contacting the skin of the patient during use. The contact portion 26 of the bladder 20 has a domed shape as shown in Fig. 2 to provide a skin contact area for contacting the surface of the skin during use. In the embodiment shown, the contact portion 26 projects outwardly from the open end 16 of the container 12 for ease of contacting the skin. In other embodiments, the contact portion 26 of the bladder 20 can be substantially flush with the open top end 16 of the container 12. The contact portion 26 of the bladder 20 is sufficiently flexible to conform to the surface of the skin when pressed against the skin. In the embodiment shown, the bladder 20 has a substantially uniform wall thickness. In other embodiments, the contact portion can be thinner or thicker than sides and bottom of the bladder to control the heat transfer and cooling effect through the contact portion.

A lid 30 is provided to fit onto the open top end 16 of the container 12 to close the container and cover the end contact portion 26 of the bladder 20. The lid 30 in the embodiment shown has a side wall 32 forming an open end 34 shown in Fig. 2 with a shape and dimension complementing the shape and dimension of the side wall 14 of the container 12. An end wall 36 is formed with the side wall 32 as a one piece assembly. The lid 30 can be provided with a thermally reflective inner surface or coating 31 to reduce heat transmission through the lid 30. In further embodiments, a reflective surface or coating 33 can be provided on an outer surface of the lid.

In the embodiment shown, the lid 30 fits over the outer surface of container 12 and is coupled to the container by a friction fit or interference fit. In the embodiment shown, the lid 30 when coupled to the container 12 is spaced a slight distance from the end contact portion 26 of the bladder 20 to inhibit heat transmission through the lid 30 to the bladder 20 and refrigerant medium 22 contained within the bladder 20.

The container 12 and lid 30 are generally made of a thermally insulating material and constructed to maintain the refrigerant medium in a frozen or cooled state for an extended period of time and preferably up to about 6-8 hours when stored at room temperature. In the embodiment of Figs. 1-3, the container 12 and lid 30 are made of a thermally insulating plastic material and have a thickness constructed to provide the desired insulating properties to maintain the refrigerant medium at a temperature to provide the cooling and desensitizing of the skin of the patient. The side wall 14 of the container 12 has sufficient thermal insulating properties so that handling of the container 12 by the patient reduces heat transmission from the hand of the patient and the outside environment through the side wall to retain the cooling temperature within the container and the refrigerant medium.

The refrigerant medium 22 within the container 12 can be a suitable composition that can be frozen by placing in a standard freezer to provide the cooling and desensitizing effect when placed in contact with the skin. In one embodiment, the refrigerant medium can be an aqueous hypertonic solution, such as an aqueous sodium chloride solution that can be placed in a freezer and frozen until ready for use. The refrigerant medium can be an aqueous solution that can include other salts to reduce or lower the freezing point of water. The refrigerant medium can be reused by refreezing the device.

The refrigerant medium can alternatively be other solutions or compositions that are able to maintain a frozen state for an extended period of time. Examples of suitable refrigerant medium or cooling medium can be a liquid or gel containing one or more compounds that is able to lower the freezing point of water. The refrigerant medium can be an aqueous mixture that can include hydroxyethylcellulose, sodium acrylate, vinyl coated silica gel, and the like. In other embodiments, the refrigerant medium can be an aqueous solution containing calcium chloride, magnesium chloride, sodium formate, potassium formate, sodium acetate, potassium acetate, propylene glycol, ethyl alcohol, propyl alcohol, sorbitol, glycerol, and mixtures thereof.

In another embodiment shown in Fig. 4, the container 40 can be made with an inner wall 42 and an outer wall 44 defining a gap 46 between the inner wall 42 and outer wall 44. The inner surfaces 48 of the inner wall 42 and the inner surface 50 of the outer wall 44 can be provided with a heat reflecting surface, finish, film, or coating 51 to reduce the heat convection through the container walls. The gap 46 between the inner wall 42 and the outer wall 44 can be filled with polymer foam or other insulation material. In the embodiment shown in Fig. 4, the gap 46 can be filled with air or inert gas to inhibit heat transmission through the side wall and bottom of the container. In a further embodiment, the gap 46 can be a vacuum or pressure below atmospheric pressure to inhibit the transfer of het through the wall and bottom of the container. The container 40 includes a bladder 20 and air gap 24 as in the previous embodiment that is received in the cavity of the container 40 to retain a refrigerant medium 22 and provide the contact end portion 26 to cooling skin of the patient.

A lid 52 of the container 40 is also constructed with an inner wall 54 and an outer wall 56 to define a gap 58 between the inner wall 54 and outer wall 56. As with the container 40, the gap 58 between the inner wall and outer wall can be filled with an insulating gas or air or can be provided with a vacuum or reduced pressure. The inner surfaces 60 of the inner wall 54 and the inner surface 62 of outer wall 56 can be provided with a thermally reflective finish or reflective coating 63.

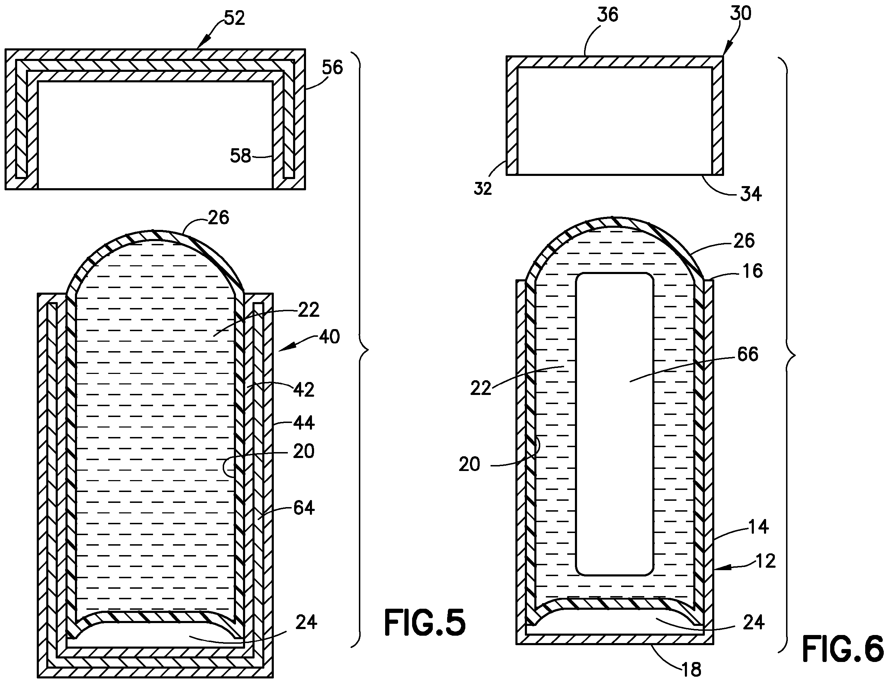

In the embodiment of Fig. 5, the gap 46 of the container 40 and the gap 58 of the lid 52 are filled with an insulating material 64, such as a foam material. The container of Fig. 5 is substantially the same as in Fig. 3 and need not be described in further detail. The bladder 20 is also substantially the same as in the previous embodiment.

In a further embodiment shown in Fig. 6, the container 12 and the bladder 20 enclose a solid core 66 within the bladder 20 to provide a greater thermal mass for retaining the refrigerant medium at the desired temperature for an extended period of time. In the embodiment shown, the core 66 has a substantially cylindrical shape complementing the shape and dimension of the bladder 20 and the container 12. The core 66 can be made of a metal such as stainless steel, aluminum or other suitable metal. The metal core 66 can be a solid material that is not reactive with the refrigerant medium.

In one embodiment, the metal core 66 includes a corrosion resistant coating. The core 66 can be contained within the bladder 20 and surrounded by the refrigerant medium 22. Typically the core 66 is completely surrounded by the refrigerant medium 22. The core in one embodiment is spaced from the skin contact portion 26 or skin contact surface.

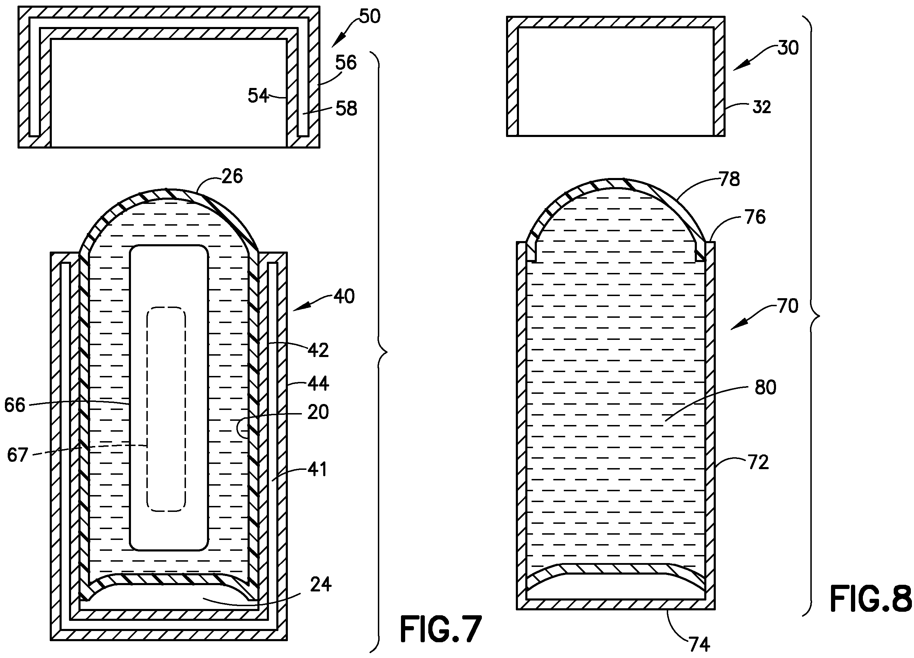

Fig. 7 shows an embodiment where the container 40 include a gap 41 or space between the inner wall 42 and the outer wall 44 providing an insulating effect. The solid core 66 is contained within the bladder 20 as in the previous embodiment.

The core 66 can have a longitudinal length extending between the open end and the bottom end of the container 12. In one embodiment, the core 66 can have a length to extend past the open end of the container as shown in Fig. 6 and Fig. 7 into the domed shaped end portion of the bladder. Typically the core is spaced from the contact area 26 so that the contact area 26 can bend and conform to the surface of the skin of the patient. The position of the core can be fixed within the bladder by a suitable attachment mechanism. In other embodiments, the core can free floating within the bladder. In other embodiments, the solid core 66 can have a length less than a length of the container to be positioned completely within the container below the open end. In further embodiments, the core can have a hollow cavity 67 as shown in Fig. 7 and enclose a refrigerant medium that is the same as or different from the refrigerant medium surrounding the core.

Fig. 8 shows another embodiment where the container 70 includes a side wall 72, a bottom wall 74, and an open top end 76. A skin contact member 78 forming a closure is attached to the container side wall 72 to enclose the cavity within the container. The refrigerant medium 80 is provided within the cavity and retained by the skin contact member 78. The container 70 can be made of a thermally insulating material as in the previous embodiments. The closure member 78 seals the container to retain the refrigerant medium 80. In one embodiment, the closure member 78 is made of a flexible polymer that is in direct contact with the refrigerant medium 80 and is able to cool the skin of the patient on contact with the skin of the patient. The flexible polymer is able to conform to the container of the skin when pressed against a target site. In other embodiments, the closure member 78 can be a rigid material such as plastic or metal.

In the embodiments shown, the refrigerant medium is contained within the bladder in a fixed position relative to the container with at least an end portion projecting from the open end of the container. In other embodiments, the bladder and refrigerant medium can be stored completely within the container and advanced to a position where the bladder containing the refrigerant medium can contact the surface of the skin of the patient.

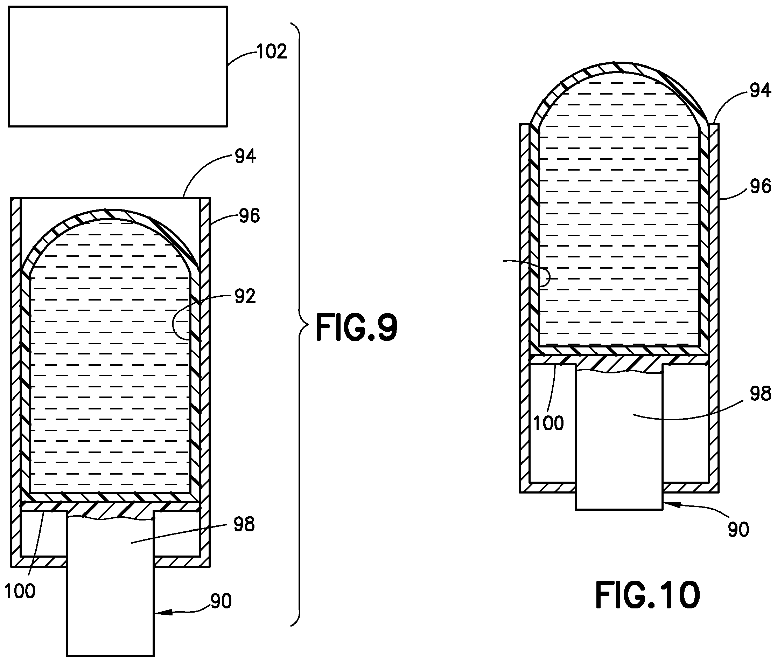

An advancing and retracting mechanism 90 can be provided to move the bladder 92 toward the open end 94 of the container 96 and retract the bladder after use. The advancing mechanism can be, for example, a threaded mechanism that moves a plunger-like member and is able to retract the bladder back into the container. In one embodiment shown in Figs. 9 and 10, the mechanism 90 can be manually operated plunger 98 coupled to a piston 100 that can be manipulated by sliding the plunger 98 toward the open end of the container to advance the bladder to a position where the bladder 20 extends from the open end of the container as shown in Fig. 10. The skin contact surface of the bladder is exposed to contact the skin of the patient to provide the cooling effect. After use, the bladder can be retracted into the container 96 as shown in Fig. 9 and covered by a lid 102. The bladder is typically retracted into the container a distance where the bladder does not contact the lid 102 to reduce the heat transfer from the refrigerant medium through the lid. Other mechanisms can be provided that are able to move the bladder to an operating position.

The device is used for the insertion of a medical device into a patient by piercing the skin of the patient and introducing the medical device subcutaneously, intravenously, intradermally, or intramuscularly. The device is particularly suitable in a method for the injection of a medication, such as insulin, into a target area of the skin. The skin contact area of the bladder is pressed against the skin in the target area of the skin for a time sufficient to cooling the skin and numb or desensitize the target area. A medical device, such as a needle or cannula then pierces the skin in the target area and the medication is introduced to the patient or fluid drawn from the patient.

The foregoing embodiments and advantages are merely exemplary and are not to be construed as limiting the scope of the present invention. The description of an exemplary embodiment of the present invention is intended to be illustrative, and not to limit the scope of the present invention. Various modifications, alternatives, and variations will be apparent to those of ordinary skill in the art, and are intended to fall within the scope of the invention. It is particularly noted that the features of different embodiments and claims may be combined with each other as long as they do not contradict each other. Accordingly, all such modifications are intended to be included within the scope of this invention as defined in the appended claims

## Claims

1. A topical cooling device (10) for cooling a target area on a surface of the skin of a patient, comprising:
a thermally insulated container (12, 40, 96) having at least one side wall (14, 44), an open end (16), a closed end, and internal cavity;
a thermally transmitting member closing said open first end (16) of said container, said thermally transmitting member (20) having a skin contact area (26) accessible through said open end (16),
wherein said thermally transmitting member includes a flexible bladder (20) forming a sealed, closed self-contained unit positioned in said container (12, 40, 96) and being flexible to conform to the inner surface of the container (12, 40, 96),
a refrigerant medium (22) within said flexible bladder (20) in said container (12, 40, 96) and in contact with said flexible bladder (20) for cooling said thermally transmitting member and the surface of the skin of a patient in contact with said thermally transmitting member, **characterized in that** said skin contact area is formed on said flexible bladder for conforming to the surface of the skin of the patient.

2. The cooling device (10) of claim 1, wherein
said refrigerant medium (22) comprises an aqueous salt solution.

3. The cooling device (10) of claim 1, further comprising
a thermally insulating lid (30, 52) closing said open end (16) of said container (12, 40, 96) and said thermally transmitting member.

4. The cooling device (10) of claim 3, wherein
said lid (30, 52) includes at least one thermally insulating layer, and wherein
said lid (30, 52) preferably includes a heat reflecting surface (63) on an inner surface of said lid (30, 52).

5. The cooling device (10) of claim 1, wherein
said container (12, 40, 96) includes an inner wall (42) and an outer wall (44) forming a gap (46) between said inner wall (42) and outer wall (44), and where said gap (46) contains a thermally insulating medium.

6. The cooling device (10) of claim 5, wherein
said thermally insulating medium comprises air or an inert thermally insulating gas and/or wherein
said thermally insulating medium comprises polymer foam.

7. The cooling device (10) of claim 5, wherein
said inner wall (42) has an inner surface with a heating reflecting surface (51) and said outer wall (44) has an inner surface with a heat reflecting surface (51).

8. The cooling device (10) of claim 1, wherein
said sealed flexible bladder (20) enclosing said refrigerant medium is received in said cavity of said container, and said flexible bladder (20) is spaced from said closed end of said container to define an air gap (24) between said flexible bladder (20) and said container (12, 40, 96).

9. The cooling device (10) of claim 8, further comprising
a thermally conducting metal core (66) received in a cavity of said bladder (20) and spaced inwardly from a side wall, a bottom wall and skin contact area of said bladder, and where said thermally conducting core is surrounded by said refrigerant medium (22), wherein
said thermally conducting core preferably comprises a metal core having a substantially cylindrical shape complementing a shape of said container (12, 40, 96), and optionally, said metal core (66) has a hollow cavity (67).

10. The cooling device (10) of claim 8, wherein
said bladder (20) is movable within said container (12, 40, 96) between a retracted position where said bladder (20) is received completely within said container (12, 40, 96) and an extended position where said skin contact area projects from said open end (16) of said container (12, 40, 96).

11. The cooling device (10) of claim 9, wherein said thermally conducting core is spaced inwardly from said contact area of said bladder (20).

12. The cooling device (10) of claim 1, wherein
said thermally transmitting member is coupled to said open end of said container (12, 40, 96) and has a substantially convex outer surface projecting from said open end (16) of said container (12, 40, 96), and
where said thermally transmitting member has surface area sufficient to contact the skin of a patient or provide a desensitizing, cooling and/or numbing effect to the skin to inhibit pain or discomfort to the patient during injection by a needle or cannula.

13. The topical cooling device (10) of claim 1, wherein
said thermally transmitting member comprises a closure closing said open first end (16) of said container (12, 40, 96) and wherein
said thermally transmitting member is preferably coupled to said side wall of said container (12, 40, 96) retaining said refrigerant medium in said container (12, 40, 96).

## Patentansprüche

1. Topische Kühlvorrichtung (10) zum Kühlen eines Zielbereichs auf einer Oberfläche der Haut eines Patienten, die aufweist:
einen thermisch isolierten Behälter (12, 40, 96) mit mindestens einer Seitenwand (14, 44), einem offenen Ende (16), einem geschlossenen Ende, und einem Innenhohlraum;
ein wärmeübertragendes Element, welches das offene erste Ende (16) des Behälters schließt, wobei das wärmeübertragende Element (20) einen Hautkontaktbereich (26) aufweist, auf den durch das offene Ende (16) zugegriffen werden kann,
wobei das wärmeübertragende Element eine flexible Blase (20) aufweist, die eine abgedichtete, geschlossene eigenständige Einheit bildet, die in dem Behälter (12, 40, 96) positioniert ist und flexibel ist, um der Innenfläche des Behälters (12, 40, 96) zu entsprechen, und
ein Kältemittel (22) innerhalb der flexiblen Blase (20) in dem Behälter (12, 40, 96) und in Kontakt mit der flexiblen Blase (20) zum Kühlen des wärmeübertagenden Elements, **dadurch gekennzeichnet, dass** der Hautkontaktbereich auf der flexiblen Blase ausgebildet ist, um der Oberfläche der Haut des Patienten zu entsprechen.

2. Kühlvorrichtung (10) nach Anspruch 1, wobei das Kältemittel (22) eine wässrige Salzlösung enthält.

3. Kühlvorrichtung (10) nach Anspruch 1, die ferner einen thermisch isolierenden Deckel (30, 52) aufweist, der das offene Ende (16) des Behälters (12, 40, 96) und das wärmeübertragene Element schließt.

4. Kühlvorrichtung (10) nach Anspruch 3, wobei
der Deckel (30, 52) mindestens eine thermisch isolierende Schicht aufweist, und wobei
der Deckel (30, 52) vorzugweise eine wärmereflektierende Oberfläche (63) auf einer Innenfläche des Deckels (30, 52) aufweist.

5. Kühlvorrichtung (10) nach Anspruch 1, wobei der Behälter (12, 40, 96) eine Innenwand (42) und eine Außenwand (44) aufweist, die einen Spalt (46) zwischen der Innenwand (42) und der Außenwand (44) bilden, und wobei der Spalt (46) ein thermisch isolierendes Medium aufweist.

6. Kühlvorrichtung (10) nach Anspruch 5, wobei
das thermisch isolierende Medium Luft oder ein inertes thermisch isolierendes Gas aufweist, und/oder wobei
das thermisch isolierende Medium Polymerschaum aufweist.

7. Kühlvorrichtung (10) nach Anspruch 5, wobei die Innenwand (42) eine Innenfläche mit einer wärmereflektierenden Oberfläche (51) aufweist und die Außenwand (44) eine Innenfläche mit einer wärmereflektierenden Oberfläche (51) aufweist.

8. Kühlvorrichtung (10) nach Anspruch 1, wobei die abgedichtete flexible Blase (20), welche das Kältemittel einschließt, in dem Hohlraum des Behälters aufgenommen ist, und wobei die flexible Blase (20) von dem geschlossenen Ende des Behälters beabstandet ist, um einen Luftspalt (24) zwischen der flexiblen Blase (20) und dem Behälter (12, 40, 96) zu definieren.

9. Kühlvorrichtung (10) nach Anspruch 8, die ferner aufweist:
einen wärmeleitenden Metallkern (66), der in einem Hohlraum der Blase (20) aufgenommen ist und von einer Seitenwand, einer unteren Wand, und einem Hautkontaktbereich der Blase nach innen beabstandet ist, und wobei der wärmeleitende Kern von dem Kältemittel (22) umgeben ist, wobei
der wärmeleitenden Kern vorzugsweise einen Metallkern mit einer im Wesentlichen zylindrischen Form aufweist, die eine Form des Behälters (12, 40, 96) ergänzt, und der Metallkern (66) optional einen Hohlraum (67) aufweist.

10. Kühlvorrichtung (10) nach Anspruch 8, wobei die Blase (20) innerhalb des Behälters (12, 40, 96) zwischen einer eingefahrenen Position, in welcher die Blase (20) vollständig in dem Behälters (12, 40, 96) aufgenommen ist, und einer ausgefahrenen Position bewegbar, in welcher der Hautkontaktbereich von dem offenen Ende (16) des Behälters (12, 40, 96) vorsteht.

11. Kühlvorrichtung (10) nach Anspruch 9, wobei der wärmeleitende Kern von dem Kontaktbereich der Blase (20) nach innen beabstandet ist.

12. Kühlvorrichtung (10) nach Anspruch 1, wobei
das wärmeübertragende Element mit dem offenen Ende des Behälters (12, 40, 96) gekoppelt ist und eine im Wesentlichen konvexe Außenfläche aufweist, die von dem offenen Ende (16) des Behälters (12, 40, 96) vorsteht, und
wobei das wärmeübertragende Element einen Flächenbereich aufweist, der ausreicht, um die Haut eines Patienten zu kontaktieren oder eine desensibilisierend, kühlende und/oder betäubende Wirkung auf die Haut auszuüben, um während der Injektion mittels einer Nadel oder Kanüle Schmerzen oder Unannehmlichkeiten für den Patienten zu vermeiden.

13. Topische Kühlvorrichtung (10) nach Anspruch 1, wobei
das wärmeübertragene Element einen Verschluss aufweist, der das offene erste Ende (16) des Behälters (12, 40, 96) verschließt, und wobei
das wärmeübertragene Element vorzugsweise mit der Seitenwand des Behälters (12, 40, 96) gekoppelt ist, wodurch das Kältemittel in dem Behälter (12, 40, 96) zurückgehalten wird.

## Revendications

1. Dispositif de refroidissement topique (10) pour refroidir une zone cible sur une surface de la peau d'un patient, comprenant :
un récipient isolé thermiquement (12, 40, 96) ayant au moins une paroi latérale (14, 44), une extrémité ouverte (16), une extrémité fermée et une cavité interne ;
un élément de transmission thermique fermant ladite première extrémité ouverte (16) dudit récipient, ledit élément de transmission thermique (20) ayant une zone de contact avec la peau (26) accessible par ladite extrémité ouverte (16),
dans lequel
ledit élément de transmission thermique comprend une poche flexible (20) formant une unité autonome fermée, scellée positionnée dans ledit récipient (12, 40, 96) et étant flexible pour se conformer à la surface interne du récipient (12, 40, 96), et
un milieu réfrigérant (22) dans ladite poche flexible (20) dans ledit récipient (12, 40, 96) et en contact avec ladite poche flexible (20) pour refroidir ledit élément de transmission thermique et la surface de la peau d'un patient en contact avec ledit élément de transmission thermique,
**caractérisé en ce que** ladite zone de contact avec la peau est formée sur ladite poche flexible pour se conformer à la surface de la peau du patient.

2. Dispositif de refroidissement (10) selon la revendication 1, dans lequel ledit milieu réfrigérant (22) comprend une solution saline aqueuse.

3. Dispositif de refroidissement (10) selon la revendication 1, comprenant en outre
un couvercle thermiquement isolant (30, 52) fermant ladite extrémité ouverte (16) dudit récipient (12, 40, 96) et ledit élément de transmission thermique.

4. Dispositif de refroidissement (10) selon la revendication 3, dans lequel
ledit couvercle (30, 52) comprend au moins une couche thermiquement isolante, et dans lequel
ledit couvercle (30, 52) comprend de préférence une surface réfléchissant la chaleur (63) sur une surface interne dudit couvercle (30, 52).

5. Dispositif de refroidissement (10) selon la revendication 1, dans lequel
ledit récipient (12, 40, 96) comprend une paroi interne (42) et une paroi externe (44) formant un intervalle (46) entre ladite paroi interne (42) et ladite paroi externe (44), et où ledit intervalle (46) contient un milieu thermiquement isolant.

6. Dispositif de refroidissement (10) selon la revendication 5, dans lequel
ledit milieu thermiquement isolant comprend de l'air ou un gaz inerte thermiquement isolant et/ou dans lequel
ledit milieu thermiquement isolant comprend une mousse polymère.

7. Dispositif de refroidissement (10) selon la revendication 5, dans lequel ladite paroi interne (42) a une surface interne avec une surface réfléchissant le chauffage (51) et ladite paroi externe (44) a une surface interne avec une surface réfléchissant la chaleur (51).

8. Dispositif de refroidissement (10) selon la revendication 1, dans lequel ladite poche flexible scellée (20) renfermant ledit milieu réfrigérant est reçue dans ladite cavité dudit récipient, et ladite poche flexible (20) est espacée de ladite extrémité fermée dudit récipient pour définir un intervalle d'air (24) entre ladite poche flexible (20) et ledit récipient (12, 40, 96).

9. Dispositif de refroidissement (10) selon la revendication 8, comprenant en outre
un noyau métallique thermoconducteur (66) reçu dans une cavité de ladite poche (20) et espacé vers l'intérieur d'une paroi latérale, d'une paroi inférieure et d'une zone de contact avec la peau de ladite poche, et où ledit noyau thermoconducteur est entouré dudit milieu réfrigérant (22), dans lequel
ledit noyau thermoconducteur comprend de préférence un noyau métallique ayant une forme sensiblement cylindrique complémentaire à une forme dudit récipient (12, 40, 96), et optionellement, ledit noyau métallique (66) a une cavité creuse (67).

10. Dispositif de refroidissement (10) selon la revendication 8, dans lequel
ladite poche (20) est mobile dans ledit récipient (12, 40, 96) entre une position rétractée où ladite poche (20) est entièrement reçue dans ledit récipient (12, 40, 96) et une position étendue où ladite zone de contact avec la peau fait saillie de ladite extrémité ouverte (16) dudit récipient (12, 40, 96).

11. Dispositif de refroidissement (10) selon la revendication 9, dans lequel ledit noyau thermoconducteur est espacé vers l'intérieur de ladite zone de contact de ladite poche (20).

12. Dispositif de refroidissement (10) selon la revendication 1, dans lequel
ledit élément de transmission thermique est couplé à ladite extrémité ouverte dudit récipient (12, 40, 96) et a une surface externe sensiblement convexe faisant saillie de ladite extrémité ouverte (16) dudit récipient (12, 40, 96), et
où ledit élément de transmission thermique a une superficie suffisante pour entrer en contact avec la peau d'un patient ou fournir un effet désensibilisant, rafraîchissant et/ou engourdissant à la peau pour inhiber la douleur ou l'inconfort du patient lors de l'injection par une aiguille ou une canule.

13. Dispositif de refroidissement topique (10) selon la revendication 1, dans lequel
ledit élément de transmission thermique comprend une fermeture fermant ladite première extrémité ouverte (16) dudit récipient (12, 40, 96) et dans lequel
ledit élément de transmission thermique est de préférence couplé à ladite paroi latérale dudit récipient (12, 40, 96) retenant ledit milieu réfrigérant dans ledit récipient (12, 40, 96).
